# EUROPÄISCHE PATENTSCHRIFT

(11) **EP 0 376 238 B1**
(45) Veröffentlichungstag und Bekanntmachung des Hinweises auf die Patenterteilung: **13.04.1994**
(21) Anmeldenummer: 89123927.9
(22) Anmeldetag: 27.12.1989
(51) Int. Cl.: C07D 211/90, C08K 5/3435

(54) **Polyalkylpiperidinsubstituierte Tetrahydropyridone und deren Verwendung**
Polyalkyl piperidine-substituted tetrahydropyridinones and their use
Tétrahydropyridinones substituées par des groupes polyalkylpipéridine et leur utilisation

(30) Priorität: 30.12.1988 DE 3844355
(43) Veröffentlichungstag der Anmeldung: 04.07.1990
(73) Patentinhaber: BASF Aktiengesellschaft, 67063 Ludwigshafen (DE)
(72) Erfinder: Aumüller, Alexander, Dr., D-6705 Deidesheim (DE); Neumann, Peter, Dr., D-6800 Mannheim 31 (DE); Trauth, Hubert, D-6724 Dudenhofen (DE)

(56) Entgegenhaltungen:
- PHOTOSTABILIZATION OF POLYMERS: Principles and Applications, J.F. Rabek, 1990, Kapitel 6, Seiten 279-356, Elsevier Applied Science, London/New York

## Beschreibung

Es ist bekannt, daß Polyalkylpiperidinderivate organische Polymere vor Zerstörung durch Licht und Wärme schützen.

Unbefriedigend ist bei den Verbindungen des Standes der Technik häufig die geringe Verträglichkeit mit Polyolefinen und anderen Kunststoffen, die Dauer der Schutzwirkung, die Eigenfarbe der Substanzen, die Neigung zur Flüchtigkeit und die thermische Zersetzung der Stabilisatoren beim Einarbeiten bei erhöhter Temperatur.

Der Erfindung liegt die Aufgabe zugrunde, neue Stabilisatoren zur Verfügung zu stellen, welche die vorstehenden Nachteile nicht aufweisen.

Die Aufgabe wird mit Hilfe der neuen Heterocyclen der Formel (I) gelöst. Dementsprechend betrifft die Erfindung polyalkylpiperidinsubstituierte Tetrahydropyridone, die in einer ihrer tautomeren Formen der allgemeinen Formel (I) 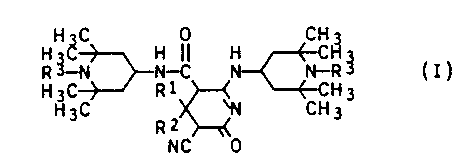
entsprechen, in der
R¹ und R² unabhängig voneinander für C₁-C₁₂-Alkyl, das gegebenenfalls durch Sauerstoff unterbrochen ist, C₅-C₆-Cycloalkyl oder C₇-C₁₂-Phenylalkyl, worin der Phenylkern unsubstituiert oder durch 1 bis 3 C₁-C₄-Alkyl, C₁-C₄-Alkoxy, Fluor, Chlor oder Brom substituiert ist, oder 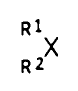
für einen 5- bis 12-gliedrigen cycloaliphatischen Ring, der gegebenenfalls durch 1 bis 4 C₁-C₄-Alkyl substituiert ist,
und
R³ für Wasserstoff, C₁-C₈-Alkyl, C₂-C₃-Cyanalkyl, C₂-C₄-Hydroxyalkyl, C₂-C₃-Aminoalkyl, C₁-C₈-Alkanoyl oder für C₇-C₁₀-Phenylalkyl stehen, worin der Phenylkern unsubstituiert oder durch 1 oder 2 C₁-C₄-Alkyl, C₁-C₄-Alkoxy, Fluor, Chlor oder Brom substituiert ist,
sowie Säureadditionssalze und Hydrate dieser Verbindungen.

Die Alkylreste R³ können linear oder verzweigt sein. Im einzelnen seien z.B. genannt: Ethyl, Propyl, n-Butyl, n-Pentyl, iso-Pentyl, n-Hexyl, n-Heptyl oder n-Octyl. Bevorzugter Alkylrest für R³ ist Methyl.

Als Alkanoylreste sind für R³ Propionyl, Butanoyl, Pentanoyl, Hexanoyl, Heptanoyl, Octanoyl, Benzoyl und insbesondere Acetyl zu nennen.

Als Phenylalkylreste für R³ sind z.B. Phenylethyl und vorzugsweise Benzyl zu nennen.

Am Phenylkern substituierte Phenylalkylreste sind beispielsweise 3- und 4-Methoxybenzyl, 3- und 4-Methoxyphenylethyl, 3- und 4-Chlorbenzyl, 3- und 4-Chlorphenylethyl, 3- und 4-Ethoxybenzyl, 3- und 4-Ethoxyphenylethyl und 3- und 4-Methylbenzyl, von denen 3- und 4-Methylbenzyl bevorzugt sind.

Cyanalkylreste für R³ sind z.B. Cyanethyl und insbesondere Cyanmethyl.

Hydroxyalkylreste für R³ sind beispielsweise 3-Hydroxypropyl, 4-Hydroxybutyl und vorzugsweise 2-Hydroxyethyl. Aminoalkylgruppen für R³ sind beispielsweise 3-Aminopropyl und vorzugsweise 2-Aminoethyl. Besonders bevorzugt für R³ ist Wasserstoff.

Die C₁-C₁₂-Alkylreste R¹ und R² können sowohl verzweigt als auch linear sein. Beispiele hierfür sind: Methyl, Ethyl, Propyl, Butyl, Pentyl, Hexyl, iso-Propyl, iso-Butyl, iso-Pentyl, tertiär-Butyl, tertiär-Amyl, Heptyl, n- und i-Octyl, Nonyl, Decyl, Undecyl und Dodecyl.

Phenylalkylreste für R¹ und R² können 7 bis 12 C-Atome enthalten, wobei der Phenylkern unsubstituiert oder durch 1 bis 3 C₁-C₄-Alkyl, C₁-C₄-Alkoxy, Fluor, Chlor oder Brom substituiert ist. Beispielhaft sind hier zu nennen Benzyl, Phenylethyl, Phenylpropyl, Phenylbutyl, 3- und 4-Methoxybenzyl, 3- und 4-Methoxyphenylethyl, 3- und 4-Chlorbenzyl, 3- und 4-Chlorphenylethyl, 3- und 4-Ethoxybenzyl, 3- und 4-Ethoxyphenylethyl, 3- und 4-Methylbenzyl und 3- und 4-Ethylbenzyl.

Als Cycloalkyl sind für R¹ und R² z.B. Cyclopentyl oder Cyclohexyl zu nennen. 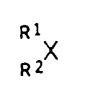
kann auch ein 5 -bis 12-gliedriger cycloaliphatischer Ring sein, wobei dieser Ring durch 1 bis 4 C₁-C₄-Alkylgruppen substituiert sein kann.

Beispiele hierfür sind Cyclopentyl, Cyclohexyl, 4-Methylcyclohexyl, 3,3,5,5-Tetramethylcyclohexyl, 4-tertiär-Butylcyclohexyl, Cycloheptyl, Cyclooctyl und Cyclododecyl.

Vorzugsweise stehen R¹ und R² für C₁-C₅-Alkyl, das linear oder verzweigt sein kann. Weiterhin sind Verbindungen bevorzugt, in denen 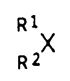
für einen Cyclopentyl-, Cyclohexyl-, Cycloheptyl- oder Cyclododecylring stehen.

Die Verbindungen (I) lassen sich durch Umsetzung von Ketonen der allgemeinen Formel (II) mit Cyanacetamiden der allgemeinen Formel (III) in einem Lösungsmittel mit einem basischen Katalysator herstellen. 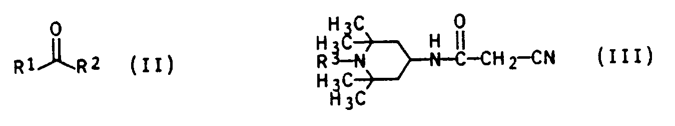
Als Lösungsmittel kommen sowohl organische Lösungsmittel als auch Wasser in Frage. Als organische Lösungsmittel sind beispielsweise Alkohole und Aromaten wie Toluol und Xylol zu nennen.

Als Lösungsmittel sind Alkohole, wie Ethanol, n-Propanol, iso-Propanol, n-Butanol und besonders Methanol zu nennen.

Bevorzugte Lösungsmittel sind Methanol und Wasser.

Als basische Katalysatoren kommen beispielsweise Amine, Alkoholate und Alkalimetallhydroxide in Betracht.

Aus der Klasse der Amine sind im einzelnen z.B. zu nennen: Triethylamin, Tributylamin, Morpholin, Pyridin, Chinolin und vor allem Piperidin.

Als Alkoholate kommen beispielsweise die Alkalimetallsalze von C₁-C₄-Alkoholen, wie Natriumethylat, Kalium-tertiär-butylat und bevorzugt Natriummethylat in Betracht.

Aus der Klasse der Alkalimetallhydroxide sind z.B. Lithiumhydroxid, Kaliumhydroxid und Natriumhydroxid zu nennen. Von diesen ist Natriumhydroxid bevorzugt.

Die erfindungsgemäßen Verbindungen (I) können in Form der freien Basen, der Hydrate oder der Salze vorliegen. Geeignete Anionen stammen z. B. von anorganischen Säuren, Carbonsäuren und Sulfonsäuren. Von den Salzen sind die der Carbonsäuren und Sulfonsäuren bevorzugt.

Als anorganische Anionen sind z.B. Chlorid, Bromid, Sulfat, Methosulfat, Tetrafluoroborat, Phosphat und Rhodanid zu nennen.

Sulfonsäure-Anionen sind z. B. Benzolsulfonat oder Tosylat.

Als Carbonsäureanionen kommen z.B. Formiat, Acetat, Propionat, Hexanoat, Cyclohexanoat, Lactat, Stearat, Dodecylbenzoat, Benzoat, Acrylat, Methacrylat, Citrat, Malonat oder Succinat sowie Anionen von Polycarbonsäuren mit bis zu etwa 3000 COOH-Gruppen in Betracht.

Die Erfindung betrifft außerdem die Verwendung von (I) als Stabilisatoren für Kunststoffe und Lacke.

Das Vermischen der erfindungsgemäßen Verbindungen (I) mit den Kunststoffen kann in allen bekannten Vorrichtungen und nach den bekannten Methoden zum Einmischen von Stabilisierungsmitteln oder anderen Zusätzen in Polymere erfolgen.

Die mit den erfindungsgemäßen Verbindungen stabilisierten Kunststoffe können gegebenenfalls noch weitere Additive enthalten, z. B. Antioxidantien, Lichtstabilisierungsmittel, Metalldesaktivatoren, antistatische Mittel, flammhemmende Mittel, Pigmente und/oder Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxyethyl]-iso cyanurat, 1,3,5-Tris-(2,6,di-methyl-3-hydroxy-4-tert.-butylbenzyl)-isocyanurat und Pentaerythrit-tetrakis-[β-(3,5-di-tert.-butyl-4-hydroxy-phenyl)-propionat] erwähnt.

Als phosphorhaltige Antioxidantien seien beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidantien und Lichtstabilisatoren, die zusammen mit den erfindungsgemäßen Verbindungen verwendet werden können, sind z. B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide oder Benzimidazolcarbonsäureanilide.

Als organische Kunststoffe, die durch die erfindungsgemäßen Verbindungen stabilisiert werden können, seien beispielsweise genannt:
Polymere von Mono- und Diolefinen, wie z. B. Polyethylen niedriger oder hoher Dichte, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polysytrol;
Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methacrylat;
ABS-, MBS- oder ähnliche Polymere;
Halogenhaltige Polymere, wie z. B. Polyvinylchlorid, Polyvinylfluorid, Polyvinylidenfluorid sowie deren Copolymere;
Polymere die sich von α,β-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen bzw. deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weiterhin können mit (I) Lacküberzüge stabilisiert werden, z. B. Industrielackierungen. Unter diesen sind Einbrennlackierungen, unter diesen wiederum Fahrzeuglackierungen, und insbesondere Zweischichtlackierungen, hervorzuheben.

Auch hier können zusätzlich die bereits aufgeführten Antioxidantien und Lichtschutzmittel mitverwendet werden.

Die erfindungsgemäßen Verbindungen können in fester oder gelöster Form dem Lack zugesetzt werden. Ihre gute Löslichkeit in Lacksystemen ist dabei von besonderem Vorteil.

Die Erfindung wird durch die nachstehenden Beispiele weiter erläutert. Die Herstellbedingungen sind in keinem Fall optimiert.

### Beispiel 1

800 ml Ethanol, 1356 g Cyanessigsäureethylester und 1860 g 2,2,6,6-Tetramethyl-4-aminopiperidin wurden 7 Stunden gekocht und anschließend im Eisbad auf 10 °C abgekühlt. Der ausgefallene Niederschlag wurde abgesaugt und mit kaltem Essigsäureethylester gewaschen. Man erhielt 1588 g der Verbindung der Formel 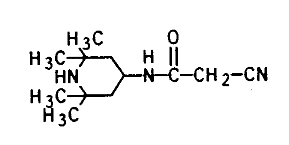
als farblosen Feststoff vom Schmp. 148 °C.

### Beispiel 2

Zu 125 ml Wasser gab man bei Raumtemperatur 66,9 g des Produkts aus Beispiel 1, 11,6 g Aceton und 5 ml Piperidin. Nach 3 h Rühren und Stehenlassen über Nacht wurde der ausgefallene Niederschlag abgesaugt, mit Wasser gewaschen und bei 100 °C im Wasserstrahlvakuum getrocknet. Man erhielt 66 g der Verbindung der Formel 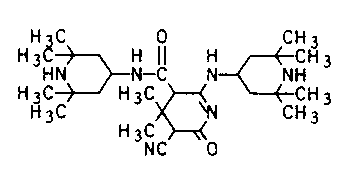
als farbloses Halbhydrat vom Schmp. 291 °C (Zers.).

| | | | | | |
|---|---|---|---|---|---|
| C₂₇H₄₆O₂N₆ · 0,5 H₂O (M 495,7) | ber.: | C 65,4 | H 9,6 | N 17,0 | O 8,1% |
| | gef.: | C 65,4 | H 9,7 | N 16,9 | O 7,8% |

### Beispiel 3

Zu 66,9 g des Produkts aus Beispiel 1 und 54 g 30 %iger methanolischer Natriummethylatlösung in 300 ml Methanol gab man 10,8 g Ethylmethylketon. Nach 48 h Stehenlassen bei Raumtemperatur wurde das Lösungsmittel im Vakuum entfernt, der Rückstand in 250 ml Wasser gelöst, mit verdünnter Salzsäure auf einen pH-Wert von 9,1 gebracht, der ausgefallene Niederschlag abgesaugt, mit Acetonitril ausgekocht und wiederum abgesaugt. Man erhielt 45 g Hydrochlorid vom Schmp. 261 °C (Zers.). Man suspendierte in Wasser, stellte mit NaOH auf einen pH-Wert von 13, saugte ab und trocknete im Vakuum bei 90 °C. Man erhielt 28 g der Verbindung der Formel 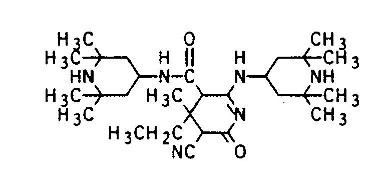
als farblosen Feststoff vom Schmp. 284 °C (Zers.).

Die Verbindung kristallisierte mit 1,5 Mol Wasser.

| | | | | | |
|---|---|---|---|---|---|
| C₂₈H₄₈O₂N₆ · 1,5 H₂O (M 527,8) | ber.: | C 63,7 | H 9,7 | N 15,9 | O 10,6% |
| | gef.: | C 63,9 | H 9,7 | N 15,8 | O 9,8% |

### Beispiel 4

66,9 g des Produkts aus Beispiel 1, 54 g 30 %ige methanolische Natriummethylatlösung und 15 g 4-Methyl-2-pentanon in 300 ml Methanol wurden wie in Beispiel 3 umgesetzt und aufgearbeitet. Nach Auskochen mit Essigester und Wasser erhielt man 16,0 g der Verbindung der Formel 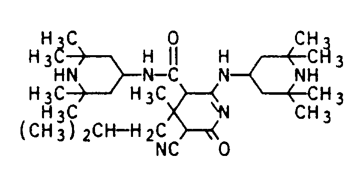
als farbloses Hydrat vom Schmp. 268 - 269 °C (Zers.).

| | | | | | |
|---|---|---|---|---|---|
| C₃₀H₅₂N₆O₂ · H₂O (M 546,8) | ber.: | C 65,9 | H 9,9 | N 15,4 | O 8,8% |
| | gef.: | C 66,0 | H 9,8 | N 15,3 | O 8,7% |

### Beispiel 5

66,9 g des Produktes aus Beispiel 1, 54 g 30 %ige methanolische Natriummethylatlösung und 29,7 g Di-n-hexylketon wurden in 300 ml Methanol 2 Tage bei Raumtemperatur stehengelassen. Nach Zugabe von 9 ml Wasser wurde 7 h gerührt, anschließend wurde das Lösungsmittel im Wasserstrahlvakuum entfernt. Der Rückstand wurde in 500 ml Wasser verrührt, mit verdünnter Salzsäure auf einen pH-Wert von 8,95 gebracht, abgesaugt und mit Wasser gewaschen. Nach Umkristallisation aus Acetonitril (Zusatz von Aktivkohle) erhielt man 18 g der Verbindung der Formel 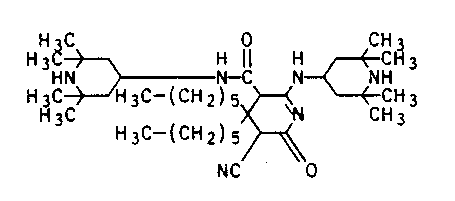
als farblosen Feststoff vom Schmp. 219 - 220 °C (Zers.).

| | | | | | |
|---|---|---|---|---|---|
| C₃₇H₆₆N₆O₂ (M 627,0) | ber.: | C 70,9 | H 10,6 | N 13,4 | O 5,1% |
| | gef.: | C 70,7 | H 10,7 | N 13,4 | O 5,1% |

### Beispiel 6

66,9 g des Produkts aus Beispiel 1, 54 g 30 %ige methanolische Natriummethylatlösung und 12,6 g Cyclopentanon wurden in 300 ml Methanol 18 h bei Raumtemperatur stehengelassen. Nach Entfernen des Lösungsmittels im Wasserstrahlvakuum wurden 400 ml Wasser und 500 ml Dichlormethan zugegeben und der ausgefallene Niederschlag abgesaugt. Nach Umkristallisation aus Acetonitril erhielt man 20 g der Verbindung der Formel 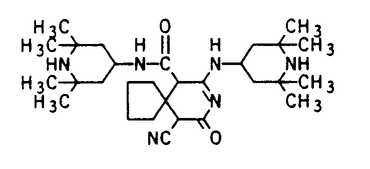
als farblosen Feststoff vom Schmp. 290 °C (Zers.).

Die Verbindung enthielt nach dem Trocknen bei 90 °C im Wasserstrahlvakuum 1,5 Mol Kristallwasser.

| | | | | | |
|---|---|---|---|---|---|
| C₂₉H₄₈N₆O₂ · 1,5 H₂O (M 531,8) | ber.: | C 64,5 | H 9,5 | N 15,6 | O 10,4% |
| | gef.: | C 64,1 | H 9,6 | N 15,6 | O 10,2% |

### Beispiel 7

147,2 g des Produkts aus Beispiel 1, 118 g 30 %ige methanolische Natriummethylatlösung und 32,3 g Cyclohexanon in 300 ml Methanol wurden wie in Beispiel 6 umgesetzt und aufgearbeitet. Nach Auskochen mit Aceton erhielt man 84 g der Verbindung der Formel 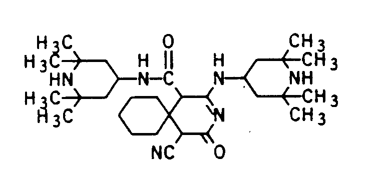
als farbloses Halbhydrat vom Schmp. > 330 °C (Zers.).

| | | | | | |
|---|---|---|---|---|---|
| C₃₀H₅₁N₆O₂ · 0,5 H₂O (M 536,8) | ber.: | C 67,1 | H 9,8 | N 15,7 | O 7,4% |
| | gef.: | C 67,1 | H 9,4 | N 15,6 | O 7,1% |

### Beispiel 8

66,9 g des Produkts aus Beispiel 1, 54 g 30 %ige methanolische Natriummethylatlösung und 16,8 g Cycloheptanon wurden in 300 ml Methanol 6 Tage stehengelassen. Nach dem Entfernen des Lösungsmittels im Wasserstrahlvakuum wurde der Rückstand in 500 ml Wasser aufgenommen, mit verdünnter Salzsäure der pH-Wert auf 9 gebracht, abgesaugt, der Rückstand mit Wasser und anschließend mit Acetonitril ausgekocht. Man erhielt 8,5 g der Verbindung der Formel 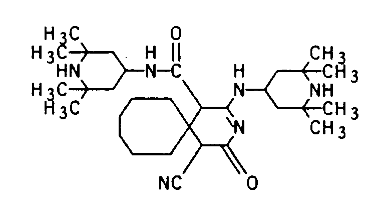
als farbloses Halbhydrat vom Schmp. 278 °C (Zers.).

| | | | | | |
|---|---|---|---|---|---|
| C₃₁H₅₂N₆O₂ · 0,5 H₂O (M 549,8) | ber.: | C 67,7 | H 9,7 | N 15,3 | O 7,3% |
| | gef.: | C 67,6 | H 9,6 | N 15,1 | O 7,1% |

### Beispiel 9

66,9 g des Produkts aus Beispiel 1, 54 g 30 %ige methanolische Natriummethylatlösung und 27,3 g Cyclododecanon wurden in 300 ml Methanol 3 Tage stehengelassen. Nach Entfernen des Lösungsmittels im Wasserstrahlvakuum wurde in 500 ml Wasser aufgenommen, der pH-Wert mit verdünnter Salzsäure auf 9 gebracht, abgesaugt und mit Wasser gewaschen. Der Rückstand wurde aus Acetonitril umkristallisiert. Man erhielt 12 g der Verbindung der Formel 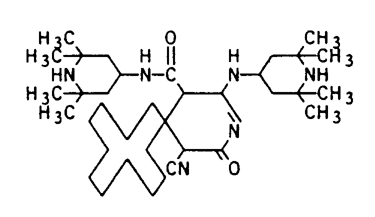
als farbloses Halbhydrat vom Schmp. 278 °C (Zers.).

| | | | | | |
|---|---|---|---|---|---|
| C₃₆H₆₂N₆O₂ · 0,5 H₂O (M 619,9) | ber.: | C 69,7 | H 10,2 | N 13,6 | O 6,4% |
| | gef.: | C 69,6 | H 10,1 | N 13,4 | O 6,2 |

### Beispiel 10

223 g des Produktess aus Beispiel 1, 58 3 g Methoxyaceton und 12 ml Piperidin wurden in 200 ml Wasser 18 h gerührt. Nach Absaugen und Trocknen erhielt man 94 g der Verbindung der Formel 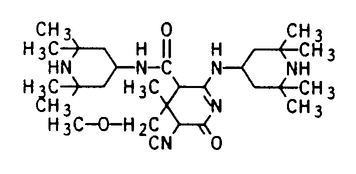
als farblosen Halbhydrat vom Schmp. 264 °C (Zers.). Das Produkt kann durch Umkristallisation am Acetonitril/Isopropanol (3:1) weiter gereinigt werden.

| | | | | | |
|---|---|---|---|---|---|
| C₂₈H₄₈N₆O₃ · 0,5 H₂O (M 525,7) | ber.: | C 64,0 | H 9,4 | N 16,0 | O 10,7% |
| | gef.: | C 63,6 | H 9,4 | N 15,9 | O 10,7% |

### Beispiel 11

37,9 g des Produkts aus Beispiel 1, 12,4 g 4-Methylcyclohexanon und 1 ml Piperidin wurden in 50 ml Methanol 8 h gerührt. Der ausgefallene Niederschlag wurde abgesaugt, mit Wasser ausgekocht, abgesaugt und getrocknet. Man erhielt 5,7 g der Verbindung der Formel 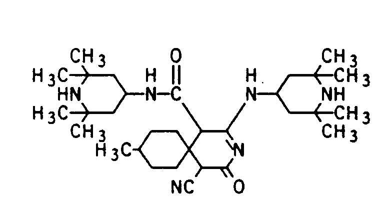
als farblosen Feststoff vom Schmp. 268 °C (Zers.), der 0,75 Mol Wasser enthält.

| | | | | | |
|---|---|---|---|---|---|
| C₃₁H₅₂N₆O₂ · 0,75 H₂O (M 554,3) | ber.: | C 67,2 | H 9,7 | N 15,2 | O 7,9% |
| | gef.: | C 67,0 | H 9,7 | N 14,9 | O 7,9% |

## Patentansprüche (Patentansprüche für folgende(n) Vertragsstaat(en): BE, CH, DE, FR, GB, IT, LI, NL)

1. Polyalkylpiperidinsubstituierte Tetrahydropyridone, die in einer ihrer tautomeren Formen der allgemeinen Formel (I) 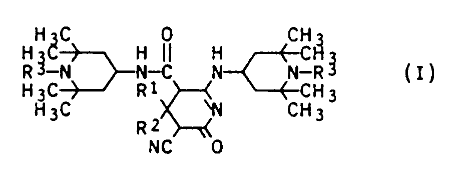 entsprechen, in der
R¹ und R² unabhängig voneinander für C₁-C₁₂-Alkyl, das gegebenenfalls durch Sauerstoff unterbrochen ist, C₅-C₆-Cycloalkyl oder C₇-C₁₂-Phenylalkyl, worin der Phenylkern unsubstituiert oder durch 1 bis 3 C₁-C₄-Alkyl, C₁-C₄-Alkoxy, Fluor, Chlor oder Brom substituiert ist, oder 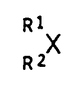 für einen 5- bis 12-gliedrigen cycloaliphatischen Ring, der gegebenenfalls durch 1 bis 4 C₁-C₄-Alkyl substituiert ist,
und
R³ für Wasserstoff, C₁-C₈-Alkyl, C₂-C₃-Cyanalkyl, C₂-C₄-Hydroxyalkyl, C₂-C₃-Aminoalkyl, C₁-C₈-Alkanoyl oder für C₇-C₁₀-Phenylalkyl stehen, worin der Phenylkern unsubstituiert oder durch 1 oder 2 C₁-C₄-Alkyl, C₁-C₄-Alkoxy, Fluor, Chlor oder Brom substituiert ist,
sowie Säureadditionssalze und Hydrate dieser Verbindungen.

2. Tetrahydropyridone gemäß Anspruch 1, in denen R¹ und R² für C₁-C₅-Alkyl stehen.

3. Tetrahydropyridone gemäß Anspruch 1, in denen 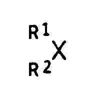 für einen 5- bis 12-gliedrigen cycloaliphatischen Ring steht.

4. Tetrahydropyridone gemäß Anspruch 1, 2 oder 3, in denen R³ für Wasserstoff steht.

5. Verwendung der Tetrahydropyridone gemäß den Ansprüchen 1 bis 4 zum Stabilisieren von Kunststoffen und Lacken.

6. Verwendung der Tetrahydropyridone gemäß den Ansprüchen 1 bis 4 zum Stabilisieren von Polyolefinen, ABS, Polyurethanen und Polyamiden.

## Patentansprüche (Patentansprüche für folgende(n) Vertragsstaat(en): ES)

1. Verwendung von polyalkylpiperidinsubstituierte Tetrahydropyridonen, die in einer ihrer tautomeren Formen der allgemeinen Formel (I) 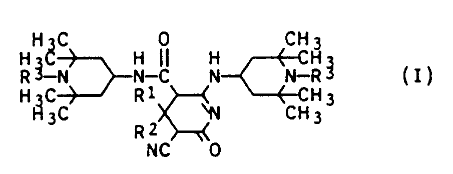 entsprechen, in der
R¹ und R² unabhängig voneinander für C₁-C₁₂-Alkyl, das gegebenenfalls durch Sauerstoff unterbrochen ist, C₅-C₆-Cycloalkyl oder C₇-C₁₂-Phenylalkyl, worin der Phenylkern unsubstituiert oder durch 1 bis 3 C₁-C₄-Alkyl, C₁-C₄-Alkoxy, Fluor, Chlor oder Brom substituiert ist, oder 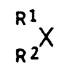 für einen 5- bis 12-gliedrigen cycloaliphatischen Ring, der gegebenenfalls durch 1 bis 4 C₁-C₄-Alkyl substituiert ist,
und
R³ für Wasserstoff, C₁-C₈-Alkyl, C₂-C₃-Cyanalkyl, C₂-C₄-Hydroxyalkyl, C₂-C₃-Aminoalkyl, C₁-C₈-Alkanoyl oder für C₇-C₁₀-Phenylalkyl stehen, worin der Phenylkern unsubstituiert oder durch 1 oder 2 C₁-C₄-Alkyl, C₁-C₄-Alkoxy, Fluor, Chlor oder Brom substituiert ist,
sowie Säureadditionssalze und Hydrate dieser Verbindungen zum Stabilisieren von Kunststoffen und Lacken.

2. Verwendung der Tetrahydropyridone gemäß Anspruch 1, wobei in der Formel R¹ und R² für C₁-C₅-Alkyl stehen.

3. Verwendung der Tetrahydropyridone gemäß Anspruch 1, wobei in der Formel 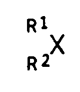 für einen 5- bis 12-gliedrigen cycloaliphatischen Ring steht.

4. Verwendung der Tetrahydropyridone gemäß Anspruch 1, 2 oder 3, wobei in der Formel R³ für Wasserstoff steht.

## Claims (Claims for the following Contracting State(s): BE, CH, DE, FR, GB, IT, LI, NL)

1. A polyalkylpiperidine-substituted tetrahydropyridone which in one of its tautomeric form conforms to the general formula (I) 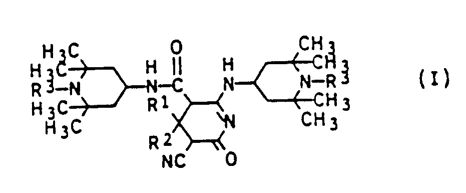 where
R¹ and R² are each independently of the other C₁-C₁₂-alkyl, which may be oxygen-interrupted, C₅-C₆-cycloalkyl or C₇-C₁₂-phenylalkyl where the phenyl is unsubstituted or monosubstituted, disubstituted or trisubstituted by C₁-C₄-alkyl, C₁-C₄-alkoxy, fluorine, chlorine or bromine, or 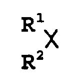 is a from 5- to 12-membered cycloaliphatic ring which may be monosubstituted, disubstituted, trisubstituted or tetrasubstituted by C₁-C₄-alkyl, and
R³ is hydrogen, C₁-C₈-alkyl, C₂-C₃-cyanoalkyl₁ C₂-C₄-hydroxyalkyl, C₂-C₃-aminoalkyl, C₁-C₈-alkanoyl or C₇-C₁₀-phenylalkyl where the phenyl is unsubstituted or monosubstituted or disubstituted by C₁-C₄-alkyl, C₁-C₄-alkoxy, fluorine, chlorine or bromine,
or an acid addition salt or hydrate thereof.

2. A tetrahydropyridone as claimed in claim 1, wherein R¹ and R² are each C₁-C₅-alkyl.

3. A tetrahydropyridone as claimed in claim 1, wherein 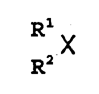 is a from 5- to 12-membered cycloaliphatic ring.

4. A tetrahydropyridone as claimed in claim 1 or 2 or 3, wherein R³ is hydrogen.

5. The use of a tetrahydropyridone as claimed in claim 1 or 2 or 3 or 4 for stabilizing plastics and coatings.

6. The use of a tetrahydropyridone as claimed in claim 1 or 2 or 3 or 4 for stabilising polyolefins, ABS, polyurethanes and polyamides.

## Claims (Claims for the following Contracting State(s): ES)

1. The use of a polyalkylpiperidine-substituted tetrahydropyridone which in one of its tautomeric form conforms to the general formula (I) 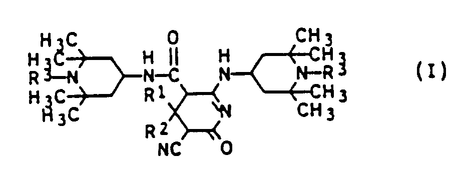 where
R¹ and R² are each independently of the other C₁-C₁₂-alkyl, which may be oxygen-interrupted, C₅-C₆-cycloalkyl or C₇-C₁₂-phenylalkyl where the phenyl is unsubstituted or monosubstituted, disubstituted or trisubstituted by C₁-C₄-alkyl, C₁-C₄-alkoxy, fluorine, chlorine or bromine, or 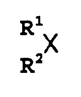 is a from 5- to 12-membered cycloaliphatic ring which may be monosubstituted, disubstituted, trisubstituted or tetrasubstituted by C₁-C₄-alkyl, and
R³ is hydrogen, C₁-C₈-alkyl, C₂-C₃-cyanoalkyl, C₂-C₄-hydroxyalkyl, C₂-C₃-aminoalkyl, C₁-C₈-alkanoyl or C₇-C₁₀-phenylalkyl where the phenyl is unsubstituted or monosubstituted or disubstituted by C₁-C₄-alkyl, C₁-C₄-alkoxy, fluorine, chlorine or bromine,
or an acid addition salt or hydrate thereof, for stabilizing plastics and coatings.

2. The use of a tetrahydropyridone as claimed in claim 1, wherein in the formula R¹ and R² are each C₁-C₅-alkyl.

3. The use of a tetrahydropyridone as claimed in claim 1, wherein in the formula 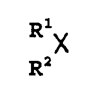 is a from 5- to 12-membered cycloaliphatic ring.

4. The use of a tetrahydropyridone as claimed in claim 1 or 2 or 3, wherein in the formula R³ is hydrogen.

## Revendications (Revendications pour l'(les) Etat(s) contractant(s) suivant(s): BE, CH, DE, FR, GB, IT, LI, NL)

1. Tétrahydropyridones à substitution polyalkylpypéridinique, qui, par leurs formes tautomériques, correspondent à la formule générale (I) : 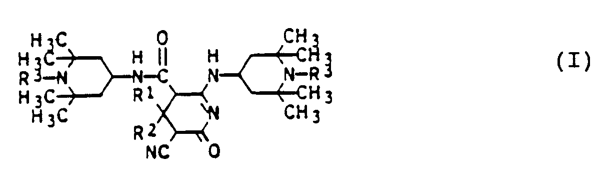 dans laquelle
R¹ et R² représentent, chacun indépendamment de l'autre, un radical alkyle en C₁-C₁₂, qui est éventuellement interrompu par de l'oxygène, un radical cycloalkyle en C₅-C₆, ou un radical phénylalkyle en C₇-C₁₂, où le noyau phényle n'est pas substitué ou est substitué par 1 à 3 radicaux alkyle en C₁-C₄, alcoxy en C₁-C₄, atomes de fluor, de chlore ou de brome, ou bien 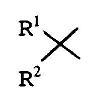 représente un noyau cycloaliphatique pentagonal à dodécagonal, éventuellement substitué par 1 à 4 radicaux alkyle en C₁-C₄,
et
R³ représente un atome d'hydrogène, un radical alkyle en C₁-C₈, cyanoalkyle en C₂-C₃, hydroxyalkyle en C₂-C₄, aminoalkyle en C₂-C₃, alcanoyle en C₁-C₈, ou un radical phénylakyle en C₇-C₁₀, où le noyau phényle n'est pas substitué ou est substitué par 1 ou 2 radicaux alkyle en C₁-C₄, alcoxy en C₁-C₄, atomes de fluor, de chlore ou de brome,
ainsi que les sels d'addition d'acides et les hydrates de ces composés.

2. Tétrahydropyridones suivant la revendication 1, dans lesquelles R¹ et R² représentent des radicaux alkyle en C₁-C₅.

3. Tétrahydropyridones suivant la revendication 1, dans lesquelles : 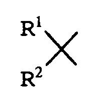 représente un noyau cycloaliphatique pentagonal à dodécagonal.

4. Tétrahydropyridones suivant la revendication 1, 2 ou 3, dans lesquelles R³ représente un atome d'hydrogène.

5. Utilisation des tétrahydropyridones suivant les revendications 1 à 4, pour la stabilisation de matières plastiques et de laques ou peintures.

6. Utilisation des tétrahydropyridones suivant les revendications 1 à 4, pour la stabilisation de polyoléfines, de l'ABS, des polyuréthannes et de polyamides.

## Revendications (Revendications pour l'(les) Etat(s) contractant(s) suivant(s): ES)

1. Utilisation de tétrahydropyridones à substitution polyalkylpypéridinique, qui, par leurs formes tautomériques, correspondent à la formule générale (I) : 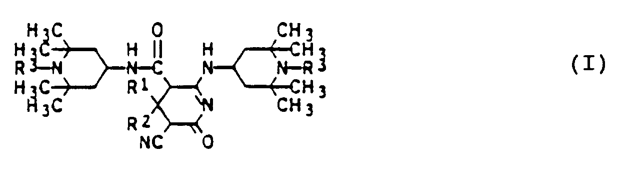 dans laquelle
R¹ et R² représentent, chacun indépendamment de l'autre, un radical alkyle en C₁-C₁₂, qui est éventuellement interrompu par de l'oxygène, un radical cycloalkyle en C₅-C₆, ou un radical phénylalkyle en C₇-C₁₂, où le noyau phényle n'est pas substitué ou est substitué par 1 à 3 radicaux alkyle en C₁-C₄, alcoxy en C₁-C₄, atomes de fluor, de chlore ou de brome, ou bien 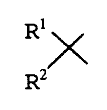 représente un noyau cycloaliphatique pentagonal à dodécagonal, éventuellement substitué par 1 à 4 radicaux alkyle en C₁-C₄,
et
R³ représente un atome d'hydrogène, un radical alkyle en C₁-C₈, cyanoalkyle en C₂-C₃, hydroxyalkyle en C₂-C₄, aminoalkyle en C₂-C₃, alcanoyle en C₁-C₈, ou un radical phénylakyle en C₇-C₁₀, où le noyau phényle n'est pas substitué ou est substitué par 1 ou 2 radicaux alkyle en C₁-C₄, alcoxy en C₁-C₄, atomes de fluor, de chlore ou de brome,
ainsi que des sels d'addition d'acides et des hydrates de ces composés pour la stabilisation de matières plastiques et de laques ou peintures.

2. Utilisation des tétrahydropyridones suivant la revendication 1, dans la formule desquelles R¹ et R² représentent des radicaux alkyle en C₁-C₅.

3. Utilisation des tétrahydropyridones suivant la revendication 1, dans la formule desquelles 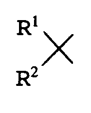 représente un noyau cycloaliphatique pentagonal à dodécagonal.

4. Utilisation des tétrahydropyridones suivant la revendication 1 ou 2, dans la formule desquelles R³ représente un atome d'hydrogène.
